# EUROPEAN PATENT APPLICATION

(11) **EP 4 783 183 A1**
(43) Date of publication of application: **29.07.2026**
(21) Application number: 26152668.5
(22) Date of filing: 19.01.2026
(51) Int. Cl.: G16H 20/40, G16H 50/20

(54) **MACHINE LEARNING ASSISTED SURGICAL DECISION SYSTEMS AND METHODS**

(30) Priority: 23.01.2025 US 202563748709 P
(71) Applicant: Mako Surgical Corp., Weston, FL 33331 (US)
(72) Inventor: ALI, Azhar, CEDAR PARK, 78616 (US); HAMPP, Emily, FAR HILLS, 07931 (US); TULIEBITZ, Jillian, LONDON, SE8 4JQ (GB); MARCHAND, Robert, WAKEFIELD, 02879 (US); GAVIN, Clark, SUBIACO, 6008 (AU)
(74) Representative: Regimbeau

(57) **Abstract**

Disclosed herein is a system for machine learning assisted surgical planning. The system can include a data acquisition module configured to collect preoperative data, intraoperative data, and postoperative data. The preoperative data can include any of imaging data, patient-reported outcome measures and functional metrics. The intraoperative data can include kinematic assessment of a knee joint. The postoperative data can include any of functional recovery metrics, radiographic data, and adverse event tracking. The system can include a machine learning model configured to process the preoperative data, intraoperative data, and postoperative data to identify patterns and relationships between surgical planning attributes and patient-specific outcomes, and generate predictive outputs including recommendations for the surgical planning attributes.

## Description

### TECHNICAL FIELD

This present disclosure relates to systems and methods for surgical planning and decision-making in orthopedic procedures, particularly knee joint surgeries. More specifically, the present disclosure is directed to the integration of machine learning algorithms with kinematic assessments to optimize intraoperative decisions and improve patient-specific surgical outcomes.

### BACKGROUND

Current approaches to knee joint surgeries, including total knee arthroplasty (TKA), rely on various intraoperative tools to assess joint stability and balance. These tools typically measure medial and lateral femorotibial gaps at specific flexion angles, such as 0 and 90 degrees. However, these discrete poses often fail to fully characterize the behavior of the soft tissue envelope throughout the full range of motion, limiting the surgeon's ability to achieve optimal joint kinematics and long-term functional outcomes.

While preoperative imaging and static intraoperative assessments provide valuable information, they do not capture the dynamic interactions of ligaments, bones, and implants during flexion and extension. Moreover, surgical decision-making remains reliant on subjective interpretation of these limited data points, which can lead to variability in outcomes among patients with unique anatomical and biomechanical profiles.

Thus, there is a need for systems and methods that effectively analyze comprehensive kinematic assessments and optimize surgical decision-making in real time.

### BRIEF SUMMARY

According to the invention, there is provided a system for machine learning assisted surgical planning as defined in claim 1, preferable embodiments thereof being defined in the dependent claims.

Disclosed herein are systems and methods to analyze kinematic assessments and optimize surgical decision-making in real time. Disclosed herein is a machine learning-assisted surgical planning system that can integrate a data acquisition module and a machine learning model. The system can collect preoperative, intraoperative, and postoperative data, including imaging data, patient-reported outcomes, functional metrics, kinematic assessments, and recovery metrics, to analyze patterns and relationships between surgical attributes and patient outcomes. The system can generate predictive outputs, such as recommendations for implant type, alignment, and soft tissue management. It can include features like real-time visualization of predictive outputs on a user interface, enabling surgeons to evaluate and toggle between surgical scenarios while visualizing quantified outcomes. The system can perform kinematic assessments under various conditions and iterative refinement of predictive outputs based on postoperative data.

Disclosed herein is a method for surgical planning using system with a ML model. The method can include collecting comprehensive data across preoperative, intraoperative, and postoperative phases and processing this data through a machine learning model to identify relationships between surgical planning attributes and patient-specific outcomes. The method can generate predictive outputs, including detailed recommendations for surgical strategies, and displays these outputs in real time during surgery via a user interface. Additional steps can include performing kinematic assessments at key flexion angles and stressed conditions, refining model predictions using postoperative data, and enabling surgeons to interact with and customize predictive scenarios through the interface.

In accordance with an aspect of the present disclosure, a system for machine learning assisted surgical planning is provided. A system according to this aspect, may include a data acquisition module configured to collect preoperative data, intraoperative data, and postoperative data. The preoperative data may include any of imaging data, patient-reported outcome measures and functional metrics. The intraoperative data may include kinematic assessment of a knee joint. The postoperative data may include any of functional recovery metrics, radiographic data, and adverse event tracking. The system may include a machine learning model configured to process the preoperative data, intraoperative data, and postoperative data to identify patterns and relationships between surgical planning attributes and patient-specific outcomes, and generate predictive outputs including recommendations for the surgical planning attributes.

Continuing in accordance with this aspect, the kinematic assessment of the knee joint may include measurement of medial and lateral laxity at multiple flexion angles under neutral and stressed conditions. The stressed conditions may include anterior drawer, posterior drawer, varus stress, and valgus stress assessments performed at multiple flexion angles. The flexion angles may include at least 0°, 30°, 45°, and 90°.

Continuing in accordance with this aspect, the machine learning model may be configured to generate predictive outputs that include recommendations for implant type, implant alignment, and soft tissue management strategies. The recommendations for implant alignment may include adjustments to tibial slope, rotational alignment, and varus or valgus positioning.

Continuing in accordance with this aspect, the system may include a user interface configured to display the predictive outputs in real time during surgery. The user interface may be configured to allow a user to evaluate surgical planning scenarios by toggling between different surgical planning attributes and visualizing quantified kinematic outcomes. The user interface may visualize predictive metrics including medial pivot ratios, anterior-posterior stability, and rotational stability.

Continuing in accordance with this aspect, the machine learning model may be configured to iteratively refine its predictive outputs by incorporating postoperative data, including radiographic data and functional recovery metrics, into its training process.

Continuing in accordance with this aspect, the preoperative data may include any of comorbidity indices, gait analysis, and bone density measurements.

Continuing in accordance with this aspect, the postoperative data may include any of length of hospital stay and discharge status.

In accordance with another aspect of the present disclosure, a method for surgical planning is provided. A method according to this aspect may include the steps of collecting preoperative data, intraoperative data, and postoperative data using a data acquisition module, processing, using a machine learning model, the preoperative data, intraoperative data, and postoperative data to identify patterns and relationships between surgical planning attributes and patient-specific outcomes, and generating predictive outputs including recommendations for the surgical planning attributes. The preoperative data may include any of imaging data, patient-reported outcome measures, and functional metrics. The intraoperative data may include kinematic assessments of a knee joint. The postoperative data may include any of functional recovery metrics, radiographic data, and adverse event tracking.

Continuing in accordance with this aspect, the kinematic assessments of the knee joint may include measurements of medial and lateral laxity at multiple flexion angles under neutral and stressed conditions. The stressed conditions may include anterior drawer, posterior drawer, varus stress, and valgus stress assessments performed at flexion angles including at least 0°, 30°, 45°, and 90°.

Continuing in accordance with this aspect, the step of generating predictive outputs may include providing recommendations for implant type, implant alignment, and soft tissue management strategies. The recommendations for implant alignment may include adjustments to tibial slope, rotational alignment, and varus or valgus positioning.

Continuing in accordance with this aspect, the method may include a step of displaying the predictive outputs in real time during surgery on a user interface. The user interface may allow a user to evaluate surgical planning scenarios by toggling between different surgical planning attributes and visualizing quantified kinematic outcomes.

Continuing in accordance with this aspect, the step of processing the data may include iteratively refining the machine learning model using postoperative data including radiographic data and functional recovery metrics to improve predictive accuracy.

### BRIEF DESCRIPTION OF THE DRAWINGS

A more complete appreciation of the subject matter of the present disclosure and the various advantages thereof can be realized by reference to the following detailed description, in which reference is made to the following accompanying drawings:
FIG. 1 shows a schematic view of a machine learning-assisted surgical planning system according to an embodiment of the present disclosure;
FIG. 2 is a schematic view of inputs for the system of FIG. 1 according to an embodiment of the present disclosure.
FIG. 3 is a schematic view of additional inputs for the system of FIG. 1 according to an embodiment of the present disclosure.
FIG. 4 is a graph of intraoperative kinematic assessments showing medial and lateral laxity data according to an embodiment of the present disclosure.
FIG. 5 is a graph showing knee laxity summary according to an embodiment of the present disclosure.
FIG. 6 is a chart showing regression analysis for final implant stability according to an embodiment of the present disclosure;
FIG. 7 is a graph showing medial pivot ratio values according to an embodiment of the present disclosure;
FIG. 8 are graphs showing medial pivot ratio for a representative patient according to an embodiment of the present disclosure;
FIG. 9 is a graph showing medial pivot ratio values according to an embodiment of the present disclosure;
FIG. 10 shows classification of native phenotypes based on coronal and sagittal alignment according to an embodiment of the present disclosure;
FIG. 11 is a graph showing coronal knee laxity measurements across four phenotypes according to an embodiment of the present disclosure;
FIG. 12 is a graph showing sagittal knee laxity data across phenotypes according to an embodiment of the present disclosure.
FIG. 13 is a graph showing anterior-posterior (AP) laxity data according to an embodiment of the present disclosure;

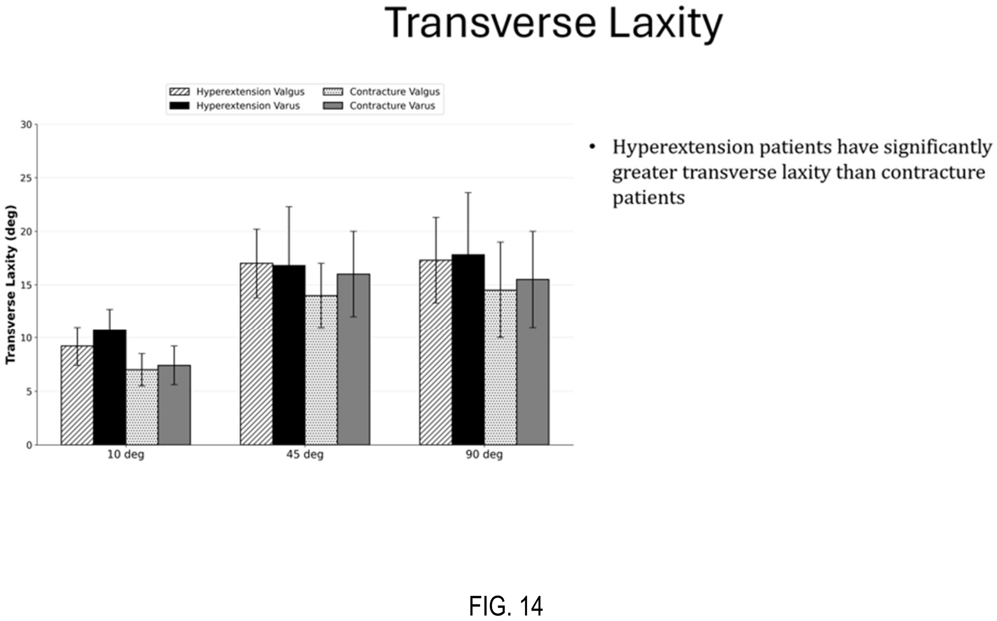

FIG. 14 is a graph showing transverse laxity measurements according to an embodiment of the present disclosure, and

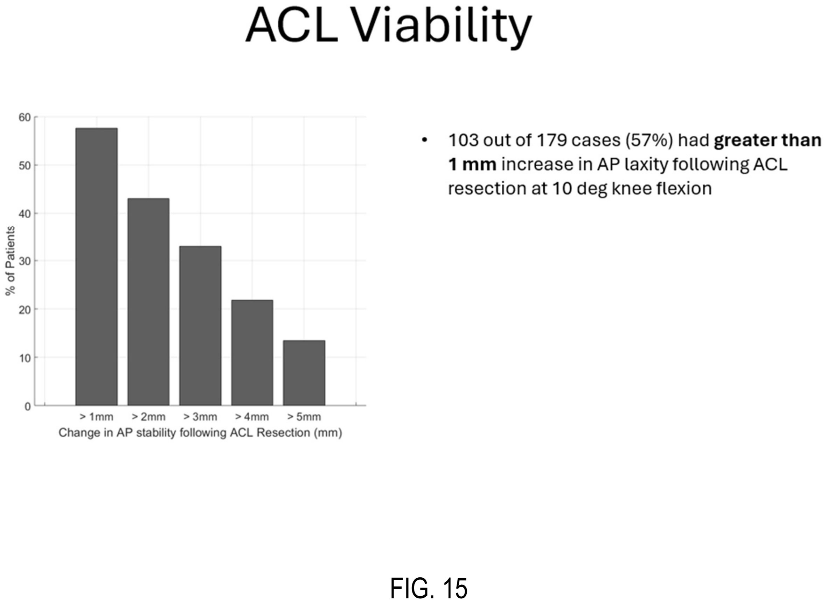

FIG. 15 is a graph showing ACL viability according to an embodiment of the present disclosure.

### DETAILED DESCRIPTION

Reference will now be made in detail to the various embodiments of the present disclosure illustrated in the accompanying drawings. Wherever possible, the same or like reference numbers will be used throughout the drawings to refer to the same or like features within a different series of numbers (e.g., 100-series, 200-series, etc.). It should be noted that the drawings are in simplified form and are not drawn to precise scale. Additionally, the term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

As used herein, the terms "HKA angle," "HKA," and "HKA alignment" will be used interchangeably and as such, unless otherwise stated, the explicit use of either term is inclusive of the other term. Similarly, the terms "joint line obliquity" and "joint line orientation" will be used interchangeably and as such, unless otherwise stated, the explicit use of either term is inclusive of the other term.

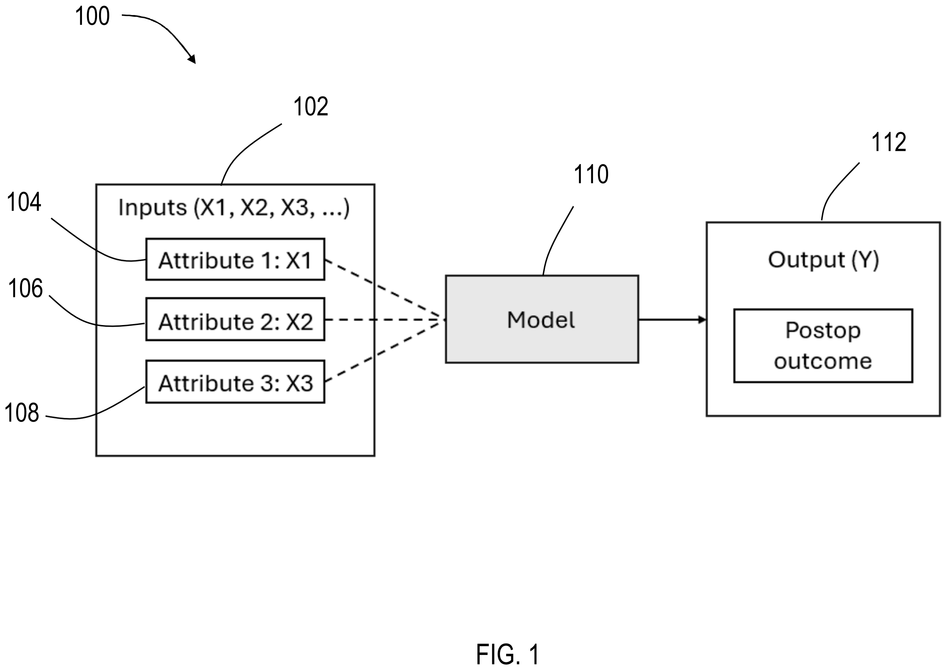

FIG. 1 shows a system 100 for machine learning-assisted surgical planning and decision-making in knee joint surgeries, particularly total knee arthroplasty (TKA). System 100 can integrate preoperative data 104, intraoperative data 106, and postoperative data 108 as input 102 to optimize surgical outcomes by analyzing patient-specific attributes and recommending surgical strategies tailored to the individual.

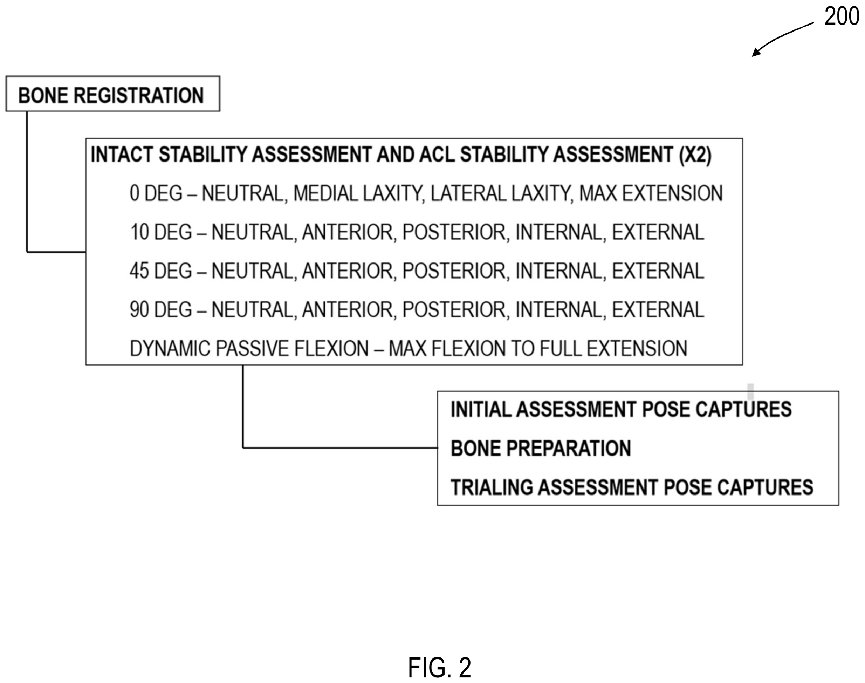

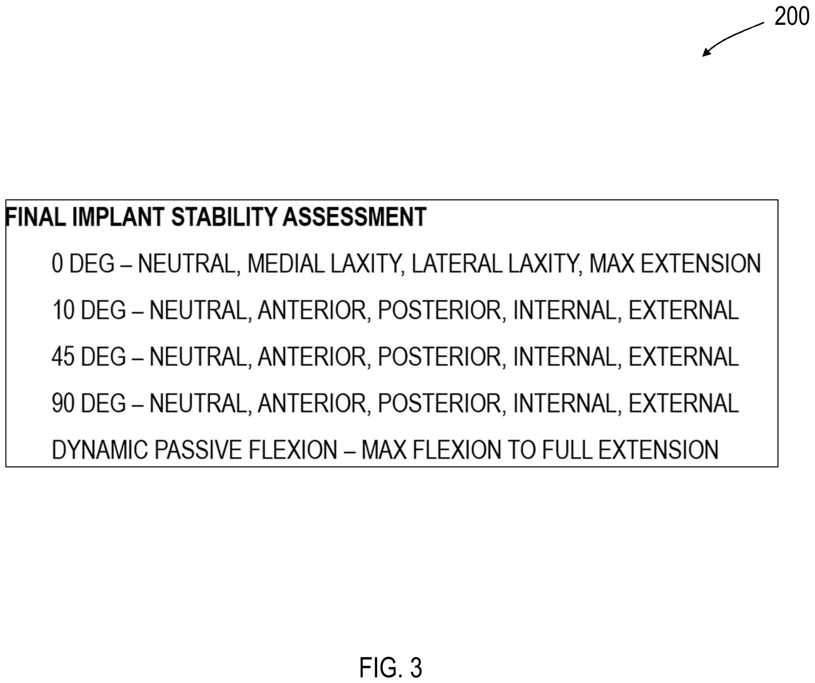

System 100 incorporates a clinical workflow 200 (FIGS. 2 and 3) that begins with preoperative data 104 acquisition, followed by intraoperative data 106 including intraoperative kinematic assessments and postoperative data 108 including postoperative outcome tracking. FIGS. 2 and 3 show specific steps of clinical workflow 200, including intact stability assessments, trialing assessments, and final implant evaluations. These steps are shown in sequential detail, highlighting the systematic capture of kinematic data, ligament stability metrics, and implant alignment parameters. During each stage, data is collected to inform a ML model 110 and refine its outputs 112 for surgical planning attributes. System 100 includes a user interface (UI) configured to display real-time predictive outputs generated by the ML model during surgery. The UI can enable surgeons to evaluate surgical planning scenarios by toggling between attributes such as implant type, alignment, and soft tissue strategies, with predictive metrics displayed as trend lines, bar graphs, or heatmaps. The interface also visualizes comparisons between preoperative predictions and intraoperative kinematic assessments, allowing surgeons to input additional intraoperative data to refine recommendations dynamically.

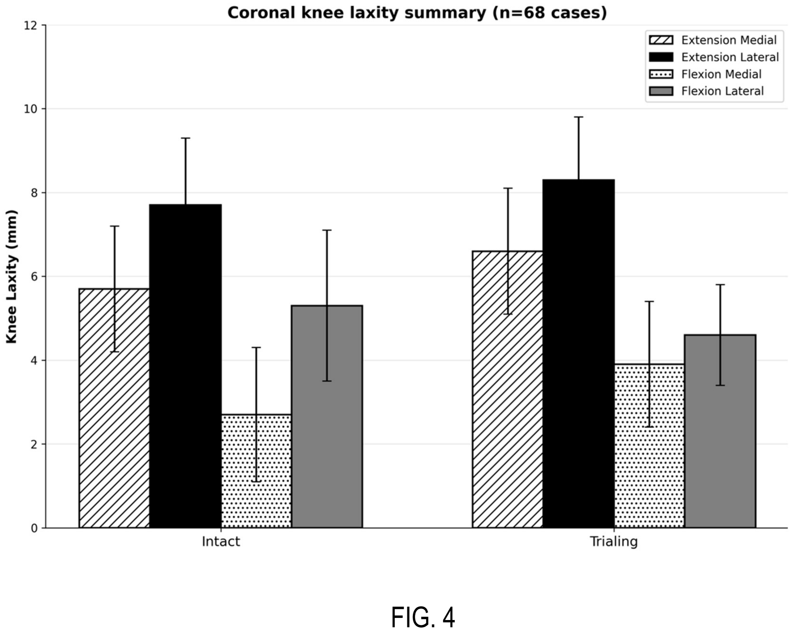

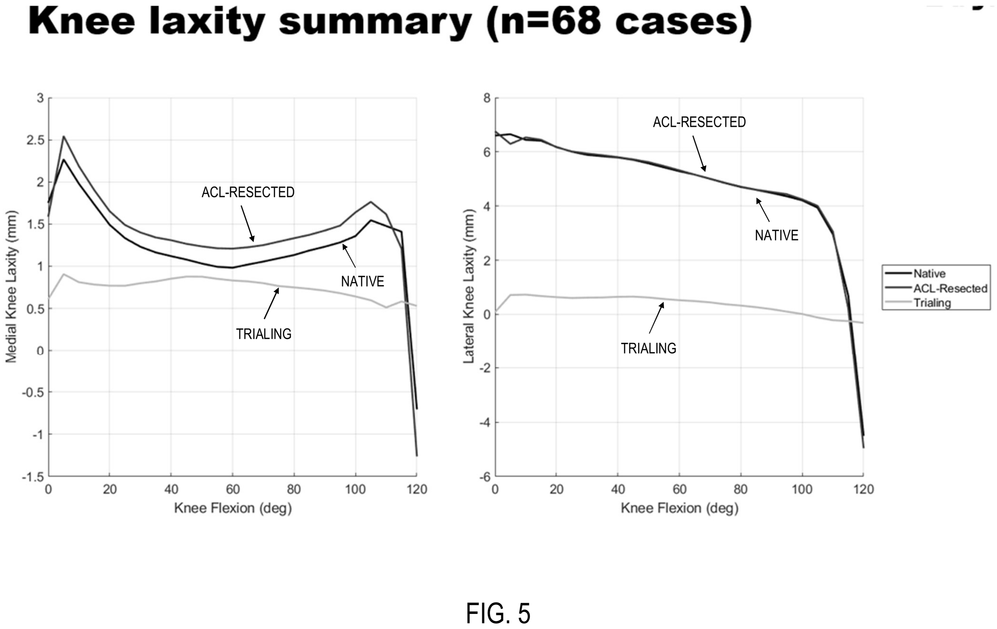

System 100 utilizes data from three distinct phases of care. Preoperative data 104 can include patient demographics, imaging modalities such as X-ray, MRI, and CT, as well as functional metrics like range of motion (ROM), gait analysis, patient-reported outcome measures (PROMs), etc. This data provides a baseline for the patient's anatomical and functional status. Intraoperative data 106, captured during surgery, can include detailed kinematic assessments performed at various flexion angles. Stability evaluations, such as anterior/posterior (AP) translation and varus/valgus stress tests, can be conducted to quantify ligament behavior under different conditions. These metrics, including medial and lateral laxity measurements, are shown in FIGS. 4 and 5. FIGS. 4 and 5 summarize knee laxity metrics measured during these assessments, including medial and lateral laxity under neutral and stressed conditions at flexion angles such as 0°, 45°, and 90°. These metrics help quantify ligament behavior and assess the functional stability of the joint. Additional intraoperative inputs can include implant positioning, alignment, and trialing results. Postoperative data 108 can include functional recovery, incorporating PROMs, ROM, radiographic evaluations, and adverse event tracking, which are used to evaluate surgical outcomes and improve the predictive accuracy of the machine learning model.

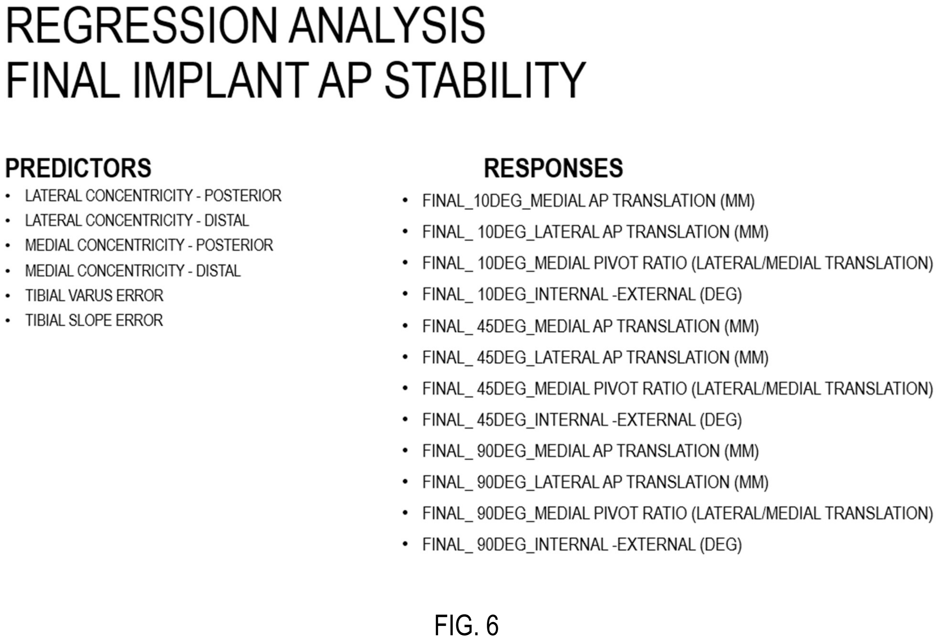

ML model 110 processes the collected data to outputs 112 which include predicted optimal surgical planning attributes, such as implant type, alignment, and soft tissue management strategies. ML model 110 can utilize regression analyses to identify relationships between intraoperative inputs and postoperative outcomes, as shown in FIG. 6. For example, ML model 110 can analyze the effects of tibial varus error or medial concentricity on anterior-posterior stability, generating recommendations to enhance implant alignment and knee balance. These predictions are provided in real time during surgery, enabling data-driven decision-making. FIG. 6 shows the results of regression analyses performed by the model, showcasing how various predictors, such as medial and lateral concentricity and tibial slope errors, influence anterior-posterior stability and other outcomes. These figures illustrate how the model evaluates these predictors and generates quantified recommendations, such as alignment adjustments or implant configurations, to optimize knee stability and balance. For instance, FIG. 6 highlights the relationship between distal medial concentricity and implant stability, and expands on how tibial slope error impacts postoperative outcomes. These insights enable the system to provide targeted recommendations during surgery.

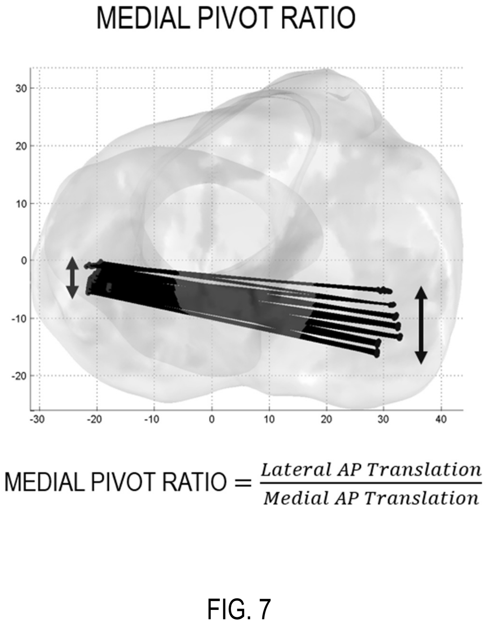

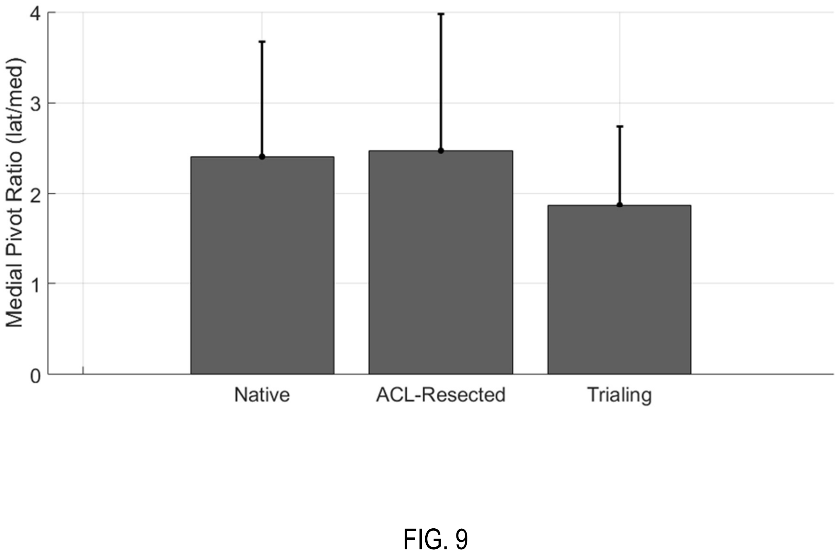

Patient-specific recommendations are generated based on the unique anatomical and biomechanical characteristics of each patient. FIGS. 7-9 show how medial pivot ratios, calculated from kinematic data, are used to compare the effectiveness of different implant designs and alignments. These figures presents data from a representative patient, illustrating the differences in medial pivot ratios across three states: native knee, ACL-resected knee, and the final implant configuration. By evaluating these states, system 100 identifies surgical plans that maximize desired outcomes, such as improved stability and ROM, while minimizing potential complications.

System 100 can receive a rich dataset collected from multiple phases of care to drive its ML model 110 and optimize surgical planning. These data inputs 102 are configured to capture detailed insights into knee joint behavior and patient-specific factors, enabling the system to provide precise, outcome-driven recommendations.

Preoperative data 104 can include an extensive set of attributes that form the baseline for predictive modeling. Key inputs can include patient-reported outcome measures (PROMs), such as the Knee Society Score (KSS), Knee Injury and Osteoarthritis Outcome Score for Joint Replacement (KOOS JR), and Visual Analog Scale (VAS), which provide subjective assessments of pain and function. Functional metrics, such as sit-to-stand performance and timed-up-and-go (TUG) tests, offer objective measures of the patient's mobility and strength. Imaging modalities, including X-rays, MRI, and CT scans, deliver a comprehensive view of bone and cartilage morphology, limb alignment, and joint health. Additional data, such as comorbidity indices (e.g., Charlson Comorbidity Index) and bone density, can further refine the preoperative profile. These inputs enable a robust predictive foundation for ML model 110.

Intraoperative data 106 can be the most dynamic and influential phase of data collection. Kinematic assessments play a central role, capturing ligament behavior and joint stability across various poses and flexion angles. As shown in FIGS.4 and 5, medial and lateral knee laxity is measured under conditions such as neutral, maximum anterior drawer, and maximum varus/valgus stresses. These measurements can be taken at critical flexion angles, including 0°, 45°, and 90°, to assess ligament functionality under varying loads. The data can also include assessments of intact ligament conditions, such as the anterior cruciate ligament (ACL) and medial collateral ligament (MCL), at their most functional ranges, such as 30° to 40° of flexion. Additionally, implant-specific details, such as type, alignment, tibial slope, and resection depth, can be recorded to evaluate their impact on knee dynamics during trialing and final implantation.

Postoperative data 108 focuses on outcomes that validate and refine ML model 110. Functional recovery metrics, such as PROMs and ROM, can be tracked to correlate surgical decisions with clinical improvements. Radiographs can provide precise measurements of implant positioning and limb alignment, while adverse event tracking ensures comprehensive monitoring of complications or deviations from expected outcomes. These inputs are pivotal for establishing long-term correlations between surgical planning attributes and patient recovery.

The diversity and granularity of the data inputs 102 enable the system to capture the complex interplay between patient anatomy, surgical execution, and postoperative recovery. FIGS. 7-9 illustrate how medial pivot ratios derived from intraoperative kinematic data provide critical insights into the effectiveness of specific implant configurations. FIG. 6 demonstrates how regression analyses leverage these inputs to identify patterns and predict surgical outcomes. By integrating and analyzing this detailed dataset, system 100 facilitates personalized, data-driven surgical decision-making.

System 100 can utilize detailed kinematic assessments to evaluate knee joint stability and ligament functionality during surgery. These assessments capture intraoperative data that inform ML model 110 and facilitate real-time surgical decision-making.

The kinematic assessment can include an evaluation of the knee in its pre-resected state, where all native ligaments are intact. These assessments can include measurements of medial and lateral laxity under neutral and stressed conditions. For example, anterior/posterior (AP) translations and varus/valgus stresses can be recorded at flexion angles such as 0°, 45°, and 90°. These measurements provide a complete picture of ligament behavior, particularly the functionality of the anterior cruciate ligament (ACL), medial collateral ligament (MCL), and lateral collateral ligament (LCL). For instance, ACL functionality is most effectively evaluated at 30° to 40° of flexion, while PCL behavior is assessed at 90° of flexion. The data collected from these evaluations can be used to establish baseline joint stability.

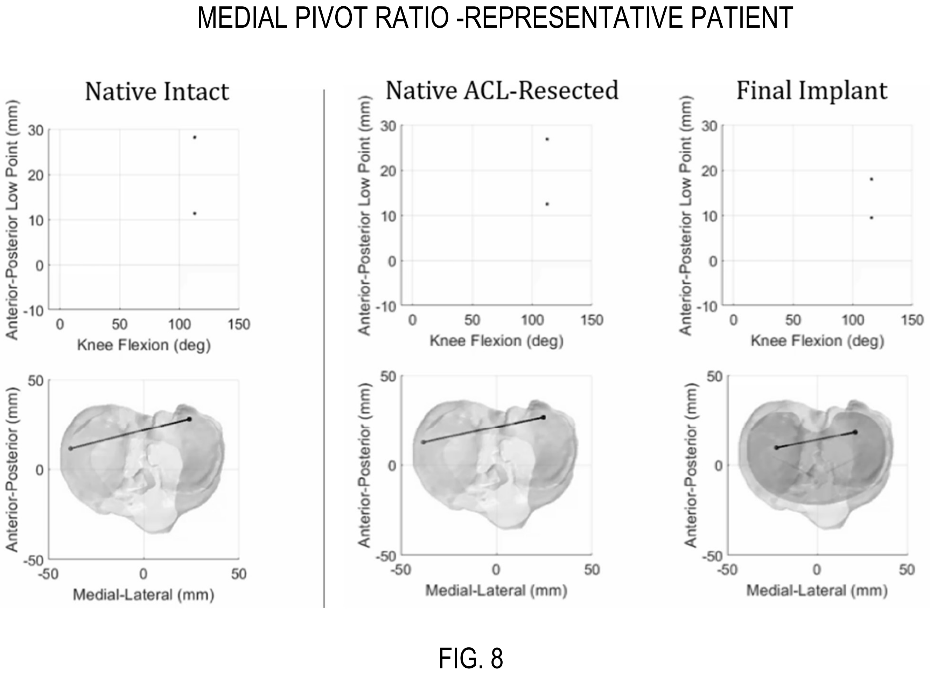

Dynamic kinematic assessments can complement static measurements by capturing the knee's motion through its full range, from deep flexion to full extension. During these assessments, the surgeon observes the behavior of the joint as it transitions between positions, allowing for the identification of instabilities or alignment issues. FIGS. 7-9 show the use of medial pivot ratios derived from these dynamic assessments to evaluate the effectiveness of specific implant designs. FIG. 8 demonstrates how the medial pivot ratio differs across states, including native, ACL-resected, and final implant configurations, providing insights into how implants restore or alter native kinematics.

During the trialing phase, kinematic assessments can be repeated to evaluate different implant configurations and alignments. Implant-specific parameters such as tibial slope, tibial varus/valgus, and rotational alignment are adjusted and assessed to ensure optimal stability and joint performance. Regression analyses can be used with the intraoperative inputs to predict surgical outcomes. For example, medial and lateral concentricity values are shown to correlate strongly with anterior-posterior stability, guiding adjustments to implant positioning and soft tissue tension.

Specialized ligament evaluations are also conducted to assess stability under specific conditions. For instance, valgus and varus stress tests at 30° flexion provide detailed insights into MCL and LCL functionality, while posterior capsule contributions are evaluated at full extension. The kinematic assessment ensures a complete analysis of joint behavior, from static measurements in the pre-resected state to dynamic evaluations during trialing. These assessments, combined with machine learning-driven insights, allow for personalized surgical adjustments tailored to the patient's anatomy and biomechanics.

ML model 110 can be trained on a multidimensional dataset collected during clinical workflow 200. ML model 110 can identify patterns and correlations within these datasets to predict the effectiveness of specific surgical planning attributes, such as implant type, alignment, and soft tissue adjustments. ML model 10 can process highly granular kinematic data, such as medial pivot ratios and ligament-specific stability metrics, to predict how specific surgical decisions will affect postoperative stability and function. Regression analyses used by ML model 110 can correlate predictors such as medial concentricity, tibial slope error, and alignment adjustments with outcomes like anterior-posterior stability and joint kinematics.

ML model 110 can adjust recommendations in real time based on intraoperative inputs. For example, data from valgus and varus stress tests at 30° flexion can be used to refine implant alignment and soft tissue tensioning recommendations. By analyzing this data in conjunction with previously trained predictors, the model suggests adjustments to ensure balanced joint mechanics and minimized ligament strain.

ML model 110 can evaluate dynamic interactions between surgical planning attributes. For example, medial pivot ratios derived from trialing assessments will allow the model to compare the impact of different implant configurations. This enables the surgeon to toggle between options such as patellar resurfacing decisions or varying levels of implant constraint, with the model providing quantified predictions for each configuration's expected outcomes.

ML model 110 can simulate the effects of multiple variables simultaneously. For example, the model can predict the combined impact of tibial alignment, implant positioning, and soft tissue releases on postoperative function, providing a holistic recommendation rather than isolated adjustments. ML model can continuously improve through iterative training. Postoperative outcomes, including radiographic data and patient-reported metrics, can be used to refine its algorithms. This ensures that the model remains adaptive to emerging clinical trends and patient-specific variability, allowing it to recommend surgical strategies with increasing precision over time.

During surgery, system 100 can process intraoperative kinematic data and provides quantified predictions for various surgical planning attributes. For example, the ML model evaluates medial pivot ratios to recommend adjustments to implant design and alignment. This data allows the surgeon to compare options such as different levels of implant constraint or varying rotational alignments, with the system predicting the functional outcomes for each scenario. These recommendations help ensure that the final implant configuration restores native joint mechanics as closely as possible.

System 100 can also toggle between multiple variables, such as tibial slope adjustments, ligament releases, and patellar resurfacing decisions, to identify the combination of attributes that maximizes desired outcomes. For example, by analyzing intraoperative medial and lateral laxity data, the system can suggest fine-tuning of ligament tension to balance the joint across its full range of motion. ML model 110 can quantify the predicted impact of each adjustment, providing the surgeon with actionable insights to guide their decisions.

System 100 can provide outputs 112 through graphical representations, such as trend lines or heatmaps. These visual tools enable surgeons to intuitively understand the implications of different surgical strategies and make informed decisions in real time.
In addition to optimizing intraoperative decisions, system 100 can support scenario-based planning. By simulating the effects of various surgical strategies, ML model 110 provides a consultation-like feature that allows surgeons to evaluate the trade-offs between different approaches. For example, system 100 may predict that a total knee implant with an MSI-type constraint in a specific alignment achieves 91% of desired kinematics, while a partial knee implant in a different configuration delivers 81%. These insights, derived from analyses such as those described above, help surgeons tailor their approach to the specific needs of the patient.

To illustrate the practical implementation of system 100, the following examples demonstrate how ML model 110, combined with intraoperative kinematic assessments, can optimize surgical outcomes for TKA. These use cases highlight the system's ability to adapt to unique patient profiles and dynamic surgical conditions.

In one scenario, a patient with severe valgus alignment presents for TKA. Preoperative imaging indicates significant lateral compartment laxity, while intraoperative assessments reveal a highly unstable lateral collateral ligament (LCL). During the trialing phase, ML model 110 analyzes medial and lateral laxity data, and suggests adjustments to implant positioning and ligament releases. The model predicts that increasing the tibial slope and fine-tuning lateral soft tissue tension will restore balance and improve stability. After implementing these adjustments, medial pivot ratios are reassessed, confirming that the final implant configuration replicates native kinematics within 90% of the desired outcome.

In another scenario, a patient undergoes ACL-resected TKA with a goal of achieving optimal anterior-posterior stability. ML model 110 evaluates intraoperative kinematic data, including anterior drawer tests at 30° and posterior drawer tests at 90° of flexion. Using predictors such as medial concentricity and tibial varus alignment, system 100 generates recommendations for implant alignment and constraint level. The model can then predict that increasing the posterior slope of the tibial implant by 2° will improve anterior stability, while maintaining medial pivot balance. After making the recommended adjustments, the trialing phase confirms that anterior-posterior stability metrics fall within the desired range.

During surgery, the surgeon can evaluate multiple implant configurations for a patient with varus alignment and compromised cartilage health. ML model 110 toggles between configurations such as a cruciate-retaining (CR) implant and a posterior-stabilized (PS) implant, using regression analyses to predict the outcomes for each option. The model determines that the PS implant provides superior anterior-posterior stability while maintaining medial-lateral balance. Additionally, the model can evaluate the impact of patellar resurfacing, recommending a specific alignment that minimizes patellar tilt and improves extensor mechanism efficiency.

In another scenario, a patient presenting with a flexion contracture limiting full knee extension undergoes a dynamic intraoperative assessment. System 100 identifies posterior capsule tension as a contributing factor. By analyzing dynamic flexion-extension data, the ML model predicts that posterior capsule releases, combined with minor adjustments to tibial slope, will restore full extension. Following these adjustments, medial and lateral laxity measurements are reevaluated, confirming balanced joint mechanics. This dynamic process ensures a personalized approach to resolving the contracture.

This figure illustrates the relationship between sagittal and coronal alignment in knee joint malalignment, categorized into four primary phenotypes: valgus flexion contracture, varus flexion contracture, valgus hyperextension, and varus hyperextension. The diagram on the left visually organizes these phenotypes based on sagittal and coronal alignment, emphasizing the interplay of these parameters in determining knee joint behavior. This classification framework is critical for understanding the biomechanical diversity across patients, which directly informs surgical planning and decision-making.

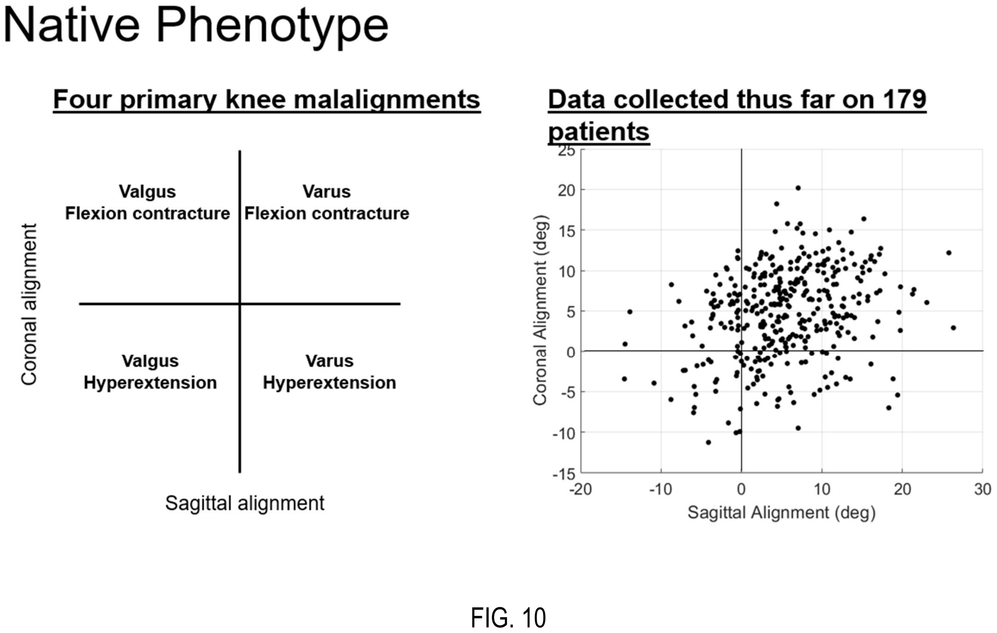

FIG. 10 shows a scatter plot representing data collected from 179 patients, showing the distribution of coronal and sagittal alignment values in a clinical setting. Each data point represents a unique patient, highlighting the variability in malalignment patterns across the population. The clustering of points provides insight into the frequency of specific malalignment types and the overlap between phenotypes, underscoring the importance of personalized surgical strategies. By classifying patients into phenotypic groups, system 100 leverages this data to refine kinematic assessments and optimize implant alignment and soft tissue adjustments. For example, a patient identified as having a valgus hyperextension phenotype may require tailored recommendations to address both lateral ligament laxity and posterior capsule tension. The use of this classification system ensures that ML model 110 accounts for these nuanced biomechanical factors when generating predictive outputs and surgical plans.

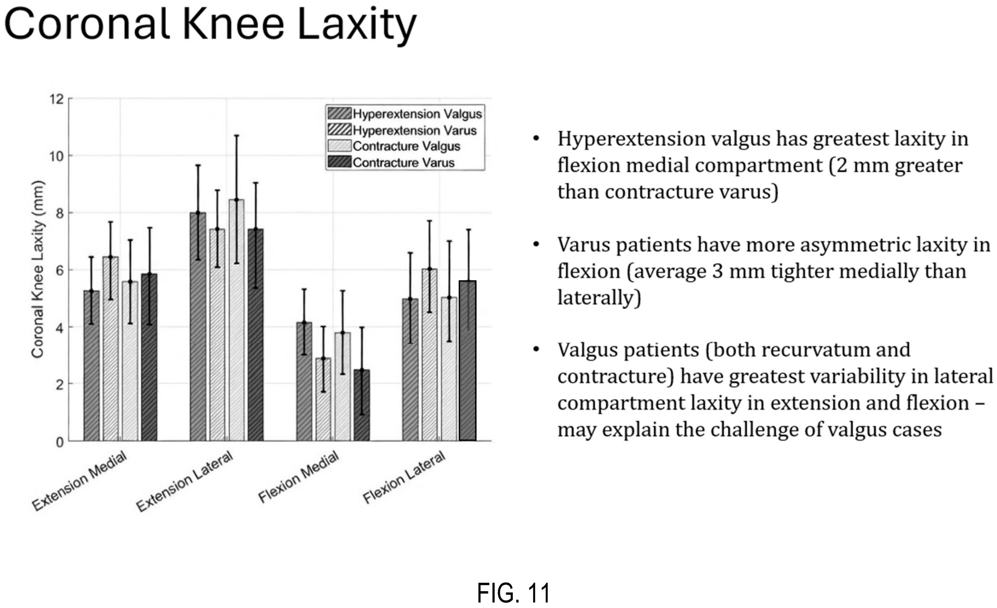

FIG. 11 provides an analysis of coronal knee laxity across different alignment phenotypes, measured in both extension and flexion states, and broken down into medial and lateral compartments. The bar graph illustrates the quantified laxity for four primary phenotypes: hyperextension valgus, hyperextension varus, contracture valgus, and contracture varus. The measurements are presented in millimeters, with error bars representing variability across the patient cohort. This data highlights key biomechanical differences between alignment phenotypes. Hyperextension valgus knees exhibit the greatest medial compartment laxity in flexion, approximately 2 mm greater than that observed in contracture varus cases. Varus patients, by contrast, show more asymmetric laxity in flexion, with an average medial tightness of 3 mm compared to lateral laxity. Valgus cases, including both recurvatum and contracture phenotypes, demonstrate significant variability in lateral compartment laxity across both extension and flexion. This variability may explain the unique challenges associated with achieving joint stability in valgus-aligned knees, particularly in surgical cases requiring soft tissue balancing.

These insights can inform ML model 110 predictions and intraoperative decisions. For instance, the ML model uses data such as medial and lateral laxity to recommend adjustments to soft tissue tension, implant alignment, and ligament releases. By incorporating the variability seen in valgus and varus phenotypes, the system can generate patient-specific surgical plans that optimize coronal balance and overall joint stability.

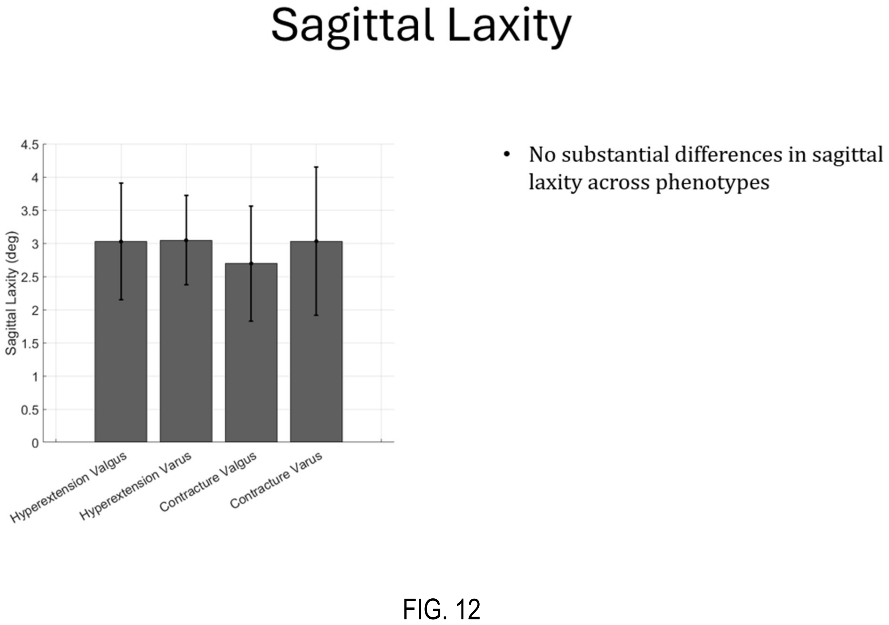

FIG. 12 shows a comparison of sagittal laxity across four primary knee alignment phenotypes: hyperextension valgus, hyperextension varus, contracture valgus, and contracture varus. Sagittal laxity is measured in degrees, with error bars representing the variability observed in the patient cohort. The results indicate no substantial differences in sagittal laxity among the alignment phenotypes, as all groups exhibit similar levels of sagittal motion within a range of approximately 2.5 to 3.5 degrees.

The lack of significant variation in sagittal laxity suggests that this parameter is less influenced by alignment phenotypes compared to coronal laxity. However, sagittal laxity remains a critical factor in evaluating the overall stability of the knee joint, particularly in cases involving anterior-posterior ligament integrity. For example, the anterior cruciate ligament (ACL) and posterior cruciate ligament (PCL) play dominant roles in controlling sagittal plane motion, and their functionality may be affected by surgical interventions such as resection or implant alignment.

FIG. 12 underscores the importance of incorporating sagittal laxity measurements into ML model 110. While the data suggests consistency across phenotypes, intraoperative measurements of sagittal motion can provide useful inputs for predicting implant performance and overall joint stability.

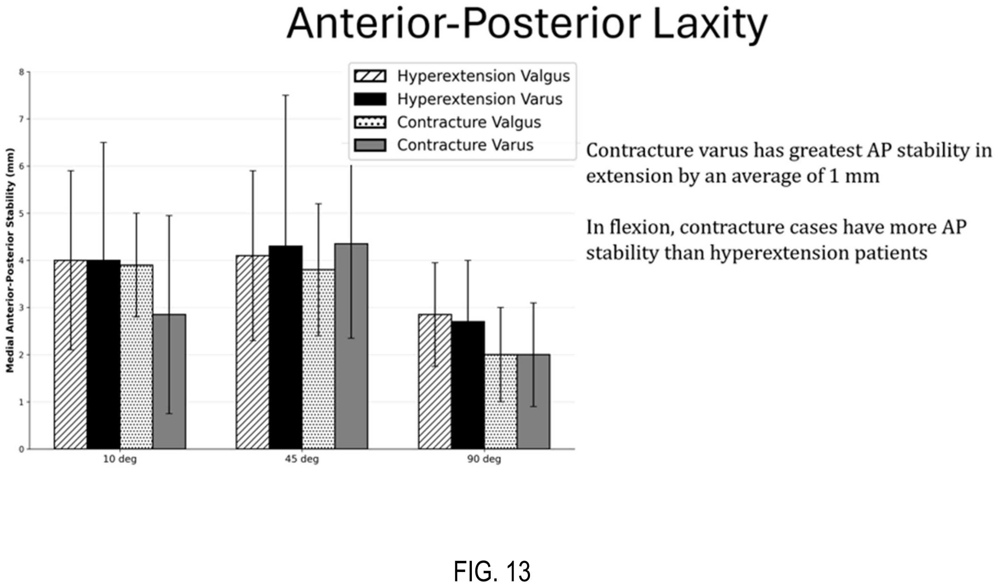

FIG. 13 shows anterior-posterior (AP) laxity measurements across different knee alignment phenotypes evaluated at three key flexion angles: 10°, 45°, and 90°. The bar graph displays the medial AP stability in millimeters, with error bars representing variability within the dataset.

The data reveals noticeable distinctions in AP stability across phenotypes. Contracture varus knees demonstrate the greatest AP stability in extension, exceeding the other phenotypes by an average of 1 mm. Conversely, in flexion, contracture cases generally exhibit greater AP stability compared to hyperextension patients. This trend underscores the biomechanical differences between flexion contractures and hyperextension malalignments, particularly in how ligament tension and joint conformity influence AP motion.

ML model 110 can use AP laxity data at different flexion angles to predict optimal adjustments to implant positioning and soft tissue management. By incorporating this variability into its predictive framework, system 100 can identify surgical strategies that restore balanced AP motion, particularly for challenging cases such as contracture varus knees.

Referring now to FIG. 12, there is shown transverse laxity measurements across the four knee alignment phenotypes: hyperextension valgus, hyperextension varus, contracture valgus, and contracture varus. The bar graph displays the transverse laxity in degrees at three flexion angles - 10°, 45°, and 90°, with error bars representing variability within the dataset.

This data highlights a significant difference between hyperextension and contracture phenotypes. Hyperextension patients exhibit greater transverse laxity compared to contracture patients across all measured flexion angles. This increased transverse motion in hyperextension knees reflects a reduced resistance to internal and external rotational stresses, which is an important consideration for restoring joint stability during surgical intervention.

The increased laxity observed in hyperextension cases informs ML models 110 predictive adjustments for implant design, alignment, and soft tissue management. For example, the ML model may recommend increasing the rotational alignment constraint for hyperextension patients to counteract excessive transverse motion. Conversely, for contracture cases with lower transverse laxity, the focus may shift to fine-tuning coronal balance or ligament tension.

FIG. 13 illustrates changes in anterior-posterior (AP) stability following ACL resection, measured at 10° of knee flexion. The bar graph shows the percentage of patients who experienced varying levels of increased AP laxity after ACL resection, with thresholds set at greater than 1 mm, 2 mm, 3 mm, 4 mm, and 5 mm.

This data reveals that 103 out of 179 cases (57%) experienced a greater than 1 mm increase in AP laxity, indicating a significant change in knee stability following ACL resection. A smaller subset of patients displayed even higher increases, with over 30% experiencing greater than 3 mm of increased laxity, emphasizing the variability in response to ACL resection across the patient cohort.

These findings highlight the role of ACL viability in maintaining AP stability and its importance for surgical planning. System 100 can use this data as a key input for ML model 110, allowing it to predict how ACL resection will affect postoperative stability and to recommend compensatory adjustments. For example, if the model predicts substantial laxity increases for a particular patient, it may suggest using a more constrained implant design or adjusting tibial slope to restore AP stability.

Furthermore, although the disclosure herein has been described with reference to particular features, it is to be understood that these features are merely illustrative of the principles and applications of the present disclosure. It is therefore to be understood that numerous modifications, including changes in the sizes of the various features described herein, may be made to the illustrative embodiments and that other arrangements may be devised without departing from the scope of the present disclosure. In this regard, the present disclosure encompasses numerous additional features in addition to those specific features set forth in the paragraphs below. Moreover, the foregoing disclosure should be taken by way of illustration rather than by way of limitation as the present invention is defined in the examples of the numbered paragraphs, which describe features in accordance with various embodiments of the invention, set forth in the claims below.

## Claims

1. A system for machine learning assisted surgical planning, comprising:
a data acquisition module configured to collect preoperative data, intraoperative data and postoperative data, the preoperative data including any of imaging data, patient-reported outcome measures and functional metrics, the intraoperative data including kinematic assessment of a knee joint, the postoperative data including any of functional recovery metrics, radiographic data, and adverse event tracking, and
a machine learning model configured to process the preoperative data, intraoperative data, and postoperative data to identify patterns and relationships between surgical planning attributes and patient-specific outcomes, and generate predictive outputs including recommendations for the surgical planning attributes.

2. The system of claim 1, wherein the kinematic assessment of the knee joint includes measurement of medial and lateral laxity at multiple flexion angles under neutral and stressed conditions.

3. The system of claim 2, wherein the stressed conditions include anterior drawer, posterior drawer, varus stress, and valgus stress assessments performed at multiple flexion angles.

4. The system of claim 3, wherein the flexion angles include at least 0°, 30°, 45°, and 90°.

5. The system of any one of claims 1 to 4, wherein the machine learning model is configured to generate predictive outputs that include recommendations for implant type, implant alignment, and soft tissue management strategies.

6. The system of claim 5, wherein the recommendations for implant alignment include adjustments to tibial slope, rotational alignment, and varus or valgus positioning.

7. The system of any one of claims 1 to 6, further comprising a user interface configured to display the predictive outputs in real time during surgery.

8. The system of claim 7, wherein the user interface is configured to allow a user to evaluate surgical planning scenarios by toggling between different surgical planning attributes and visualizing quantified kinematic outcomes.

9. The system of claim 7 or claim 8, wherein the user interface visualizes predictive metrics including medial pivot ratios, anterior-posterior stability, and rotational stability.

10. The system of any one of claims 1 to 9, wherein the machine learning model is configured to iteratively refine its predictive outputs by incorporating postoperative data, including radiographic data and functional recovery metrics, into its training process.

11. The system of any one of claims 1 to 10, wherein the preoperative data includes any of comorbidity indices, gait analysis, and bone density measurements.

12. The system of any one of claims 1 to 11, wherein the postoperative data includes any of length of hospital stay and discharge status.

13. The system of any one of claims 1 to 12, wherein the machine learning model is configured to calculate medial pivot ratios from the intraoperative data and compare the medial pivot ratios across different states including native knee, ACL-resected knee, and final implant configuration to optimize implant selection.

14. The system of any one of claims 1 to 13, wherein the machine learning model is configured to classify patients into phenotype groups based on coronal and sagittal alignment, the phenotype groups including valgus flexion contracture, varus flexion contracture, valgus hyperextension, and varus hyperextension.

15. The system of any one of claims 1 to 14, wherein the machine learning model utilizes regression analysis to correlate predictors including medial concentricity, lateral concentricity, and tibial slope error with anterior-posterior stability outcomes.
